(19) Europäisches Patentamt
European Patent Office
Office européen des brevets

(11) Veröffentlichungsnummer: **0 043 573**
**B1**

(12) # EUROPÄISCHE PATENTSCHRIFT

(45) Veröffentlichungstag der Patentschrift: **31.08.83**

(51) Int. Cl.³: **C 07 D 263/58,** C 07 D 277/68

(21) Anmeldenummer: **81105184.6**

(22) Anmeldetag: **04.07.81**

(54) **Verfahren zur Herstellung von 2,6-Dichlorbenzoxazol bzw. 2,6-Dichlorbenzthiazol.**

(30) Priorität: **09.07.80 DE 3025910**

(43) Veröffentlichungstag der Anmeldung: **13.01.82 Patentblatt 82/2**

(45) Bekanntmachung des Hinweises auf die Patenterteilung: **31.08.83 Patentblatt 83/35**

(84) Benannte Vertragsstaaten: **CH DE FR GB IT LI NL**

(56) Entgegenhaltungen:
**DE-A-1 670 221**
**DE-A-2 758 002**
**DE-A-2 830 066**
**DE-B-1 164 413**

(73) Patentinhaber: **HOECHST AKTIENGESELLSCHAFT, Postfach 80 03 20, D-6230 Frankfurt am Main 80 (DE)**

(72) Erfinder: **Knorr, Harald, Dr., Völklinger Weg 34, D-6000 Frankfurt am Main 71 (DE)**
Erfinder: **Handte, Reinhard, Dr., Breckenheimer Strasse 45, D-6238 Hofheim am Taunus (DE)**
Erfinder: **Willms, Lothar, Dr., Grüner Weg 4, D-5463 Unkel (DE)**
Erfinder: **Tammer, Thomas, Dr., Höchster Strasse 22, D-6238 Hofheim am Taunus (DE)**

EP 0 043 573 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von 2,6-Dichlorbenzoxazol bzw. 2,6-Dichlorbenzthiazol

Es ist bekannt, dass man 2-Chlorbenzthiazole und -oxazole durch direkte Chlorierung der entsprechenden 2-Mercaptobenzazole herstellen kann [DE-OS 1 670 453, J. org. Chem. 23, 1500 (1958)]. Hierbei wird im Falle des Thiazols in Gegenwart katalytisch wirksamer Mengen N-substituierter Carbonsäureamide, wie z.B. Dimethylformamid, bei erhöhten Temperaturen gearbeitet, und man erhält das 2-Chlorbenzthiazol in einer Reinausbeute von 87% d.Th.

Zur Herstellung des Oxazols wird ebenfalls in einem inerten Lösungsmittel, jedoch bei niedriger Temperatur und ohne Zusatz eines Katalysators gearbeitet; die Ausbeute an 2-Chlorbenzoxazol beträgt 82% der Theorie. Will man nun auf die gleiche Weise, jedoch ausgehend von 6-Chlor-2-mercaptobenzthiazol oder 6-Chlor-2-mercaptobenzoxazol, 2,6-Dichlorbenzthiazol oder 2,6-Dichlorbenzoxazol synthetisieren, welche wertvolle Zwischenprodukte, z.B. für die Herstellung von Pflanzenschutzwirkstoffen darstellen (DE-OS 2 640 730), so muss man feststellen, dass man hier nur erheblich niedrigere Ausbeuten erreichen kann und eine grosse Menge undestillierbarer Rückstände entstehen.

Der Erfindung lag daher die Aufgabe zugrunde, die Ausbeuten bei der Herstellung von 2,6-Dichlorbenzthiazol und 2,6-Dichlorbenzoxazol zu verbessern und die Bildung von nicht wiederverwendbaren Nebenprodukten zu vermindern.

Es wurde gefunden, dass dies möglich ist, wenn man anstelle der freien 6-Chlor-2-mercapto-benzazole deren Kalium- oder Natriumsalze chloriert und diese dabei in speziellen inerten Lösungsmitteln suspendiert.

Die Erfindung betrifft daher ein Verfahren zur Herstellung von 2,6-Dichlorbenzoxazol oder 2,6-Dichlorbenzthiazol der Formel

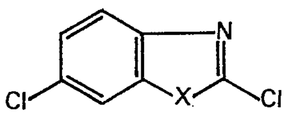

X = O, S

welches dadurch gekennzeichnet ist, dass man das Kalium- oder Natriumsalz des 6-Chlor-2-mercapto-benzoxazols oder -benzthiazols in Anwesenheit eines halogenierten aliphatischen oder aromatischen Kohlenwasserstoffs als Suspendiermittel, gegebenenfalls in Gegenwart eines Katalysators, der Chlorierung unterwirft.

Nach der erfindungsgemässen Arbeitsweise werden Ausbeuten von 90% oder mehr an den gewünschten Verfahrensprodukten erhalten. Entsprechend gering sind die Rückstandsmengen.

Der glatte Verlauf der Reaktion war in mehrfacher Hinsicht nicht vorhersehbar. So ist es überraschend, dass die Reaktion überhaupt abläuft, da die Alkalisalze der freien Mercaptoverbindungen in den zu verwendenden halogenierten Kohlenwasserstoffen praktisch unlöslich sind. Zum anderen war bekannt, dass beim Chlorieren von Benzthiazol- und Benzoxazol-mercaptiden in Lösung nur die Disulfide entstehen (DE-OS 2 800 462).

Das erfindungsgemässe Verfahren gestattet es auch von rohen 6-Chlor-benzthiazol- bzw. 6-Chlor-benzoxazol-2-alkalimercaptiden, die als Zwischenprodukte anfallen, auszugehen, ohne dass eine wesentliche Ausbeuteminderung in Kauf genommen werden muss.

Die Chlorierung wird im Temperaturbereich zwischen −20 und +150°C durchgeführt. Für die Chlorierung des Thiazols werden etwas höhere Temperaturen benötigt (>20°C, vorzugsweise 80–100°C) als für die des Oxazols, die zwischen −10 und 100°C, vorzugsweise zwichen 20 und 60°C, bei der sich spontan einstellenden Temperatur durchgeführt wird. Vorteilhaft wird bei der Chlorierung des Thiazols so verfahren, dass ein Teil der benötigten Chlormenge bei Temperaturen von 20 bis 80°C eingeleitet wird, bis sich das Sulfenylchlorid gebildet hat, worauf man die Temperaturen erhöht und weiterchloriert. Es ist auch möglich, bei der höheren Temperatur das gesamte Chlor einzuleiten. Beim Oxazol erfordert die Chlorierung des sich intermediär bildenden Sulfenylchlorids keine erhöhten Temperaturen.

Die benötigte Chlormenge beträgt ca. 2,3 bis 3 Mol pro Mol eingesetztes Alkalimercaptid, wenn das Chlor drucklos in die Reaktionsmischung eingeleitet wird. Arbeitet man im geschlossenen System, d.h. unter weitestgehendem Ausschluss von Chlorverlusten, so lässt sich die Chlormenge auf 2,3 bis ca. 2,0 Mol reduzieren.

Als Lösungsmittel werden halogenierte Kohlenwasserstoffe wie z.B. Tetrachlorkohlenstoff, Tetrachlorethan, chlorierte Benzole, vorwiegend jedoch Chlorbenzol und o-Dichlorbenzol und insbesondere das letztgenannte verwendet. Die Lösungsmittelmenge wird im allgemeinen so gewählt, dass eine noch rührbare Suspension entsteht. Es ist jedoch auch möglich, weniger Lösungsmittel zu verwenden und dann im geschlossenen System Chlor unter Druck in die Suspension einzuleiten.

Gegebenenfalls können zur Beschleunigung der Reaktion katalytische Mengen eines N-substituierten Carbonsäureamids, z.B. Dimethylformamid, zugesetzt werden.

Das bei der Reaktion sich bildende Schwefeldichlorid wird nach Beendigung der Umsetzung abdestilliert, gegebenenfalls auch in Mischung mit dem Lösungsmittel, wobei letzteres wiederverwendet werden kann.

Das bei der Chlorierung anfallende Alkalichlorid kann durch Filtration abgetrennt oder aber, bei der Chlorierung des Thiazols, nach vorheriger Entfernung von Schwefeldichlorid mit Wasser extrahiert werden.

Die als Ausgangsmaterial benötigten Alkalimercaptide des 6-Chlor-2-mercapto-benzoxazols und -benzthiazols sind z.B. nach dem in der deutschen Patentanmeldung P 30 08 225 be-

schriebenen Verfahren zugänglich bzw. lassen sich durch Umsetzung von 5-Chlor-2-aminophenol mit Alkalixanthaten herstellen.

Das erfindungsgemässe Verfahren wird z.B. in der Weise durchgeführt, dass man das Mercaptid (im Falle des 6-Chlor-2-mercaptobenzoxazols insbesondere das Kaliumsalz) im Lösungsmittel suspendiert und zunächst bei Raumtemperatur, gegebenenfalls unter Kühlung und gegebenenfalls in Gegenwart von katalytisch wirksamen Mengen Dimethylformamid (0,1 bis 5 Gew.-%, bezogen auf das Mercaptid) 1,0 bis 1,05 Mol Chlor pro Mol Mercaptid einleitet. Dann erhitzt man beim Thiazol auf 80 bis 100°C und leitet bei dieser Temperatur weitere 1,3 bis 2,0 Mol Chlor ein; beim Oxazol erfolgt die weitere Chlorzugabe bei Raumtemperatur. Hierauf destilliert man das gebildete Schwefeldichlorid ab, entfernt das Alkalichlorid durch Filtration bzw. durch Wasserextraktion und beseitigt schliesslich die flüchtigen Bestandteile (Lösungsmittel) durch Abdestillieren bei Normaldruck. Durch Destillation im Vakuum erhält man dann das 2,6-Dichlorbenzazol in sehr feinem Zustand. Auf eine weitere Reinigung des Reaktionsprodukts kann verzichtet werden.

Die folgenden Beispiele sollen die Erfindung näher erläutern.

Beispiel 1

240 g (1 Mol) 2-Kaliummercapto-6-chlorbenzthiazol werden in 800 ml Tetrachlorethan suspendiert und mit 3,5 g Dimethylformamid versetzt. Man leitet bei Raumtemperatur 1,0 Mol Chlor und bei 85 bis 90°C 1,4 Mol Chlor ein. Dann wird das Schwefeldichlorid zusammen mit etwas Tetrachlorethan abdestilliert und das Kaliumchlorid abfiltriert, welches man mit 200 ml Tetrachlorethan nachwäscht. Nach dem Abdestillieren des Tetrachlorethans bei Normaldruck erhält man durch Vakuumdestillation (bei 1,3 mbar) 192,1 g 2,6-Dichlorbenzthiazol (GC-Reinheit 99,6%ig) entsprechend einer Reinausbeute von 93,8% d.Th. Schmelzpunkt 96°C.

Beispiel 2

Es wird wie in Beispiel 1 beschrieben gearbeitet. Man leitet jedoch 3,0 Mol Chlor ein. Als Lösungsmittel wird Chlorbenzol verwendet.

Ausbeute: 191,1 g 2,6-Dichlorbenzthiazol (GC-Reinheit 99,9%ig) entsprechend einer Reinausbeute von 93,7% d.Th. Schmelzpunkt 96°C.

Beispiel 3

240 g (1 Mol) 2-Kaliummercapto-6-chlorbenzthiazol (98%ig) werden in 800 ml Chlorbenzol suspendiert. Man gibt 3,5 g Dimethylformamid zu und leitet zunächst bei Raumtemperatur 1 Mol Chlor und dann bei 85 bis 90°C nochmals 2 Mol Chlor ein. Danach destilliert man das entstandene Schwefeldichlorid zusammen mit etwas Chlorbenzol ab (150,9 g) und gibt 200 ml Wasser bei ca. 60°C zu. Die wässrige Phase wird abgelassen, worauf man aus der organischen Phase das Lösungsmittel bei Normaldruck abdestilliert. Der Rückstand wird im Vakuum bei 1,3 mbar destilliert, wobei 187,4 g 2,6-Dichlorbenzthiazol anfallen (GC: 98%ig), entsprechend einer Reinausbeute von 92,0% d.Th. Schmelzpunkt 95°C.

Beispiel 4

Man arbeitet wie in Beispiel 1, jedoch ohne den Zusatz von Dimethylformamid und erhält 190,6 g (93,4% d.Th.) 2,6-Dibenzthiazol.

Beispiel 5

223,5 g (1 Mol) 6-Chlor-2-kaliummercaptobenzoxazol werden in 500 ml Chlorbenzol suspendiert und innerhalb von 3 Stunden bei einer Temperatur von ca. 25°C mit 180 g (2,5 Mol) Chlorgas versetzt. Es wird 12 Stunden bei Raumtemperatur nachgerührt, überschüssiges Chlor wird mit Stickstoff ausgeblasen. Das ausgefallene Kaliumchlorid wird abgesaugt, mit ca. 200 ml Chlorbenzol nachgewaschen. Das Filtrat wird anschliessend destilliert. Nachdem Schwefeldichlorid und Chlorbenzol abdestilliert sind, erhält man 171 g (91% d.Th.) an 2,6-Dichlorbenzoxazol mit einem Fp. von 49–51°C und einem Kp. von 124–128,5°C bei ca. 25 mbar.

Beispiel 6 (Vergleich)

202 g (1 Mol) 6-Chlor-2-mercapto-benzthiazol und 3,5 g Dimethylformamid werden in 1000 ml Tetrachlorethan suspendiert, und es wird bei Raumtemperatur zunächst 1,05 Mol Chlor, dann bei 85°C 1,4 Mol Chlor eingeleitet. Man destilliert das Schwefeldichlorid und das Tetrachlorethan bei Normaldruck ab und erhält dann im Vakuum von 1,3 mbar bei einer Abgangstemperatur von ca. 123°C 167,6 g 2,6-Dichlorbenzthiazol entsprechend 82,1% d.Th.

**Patentansprüche**

1. Verfahren zur Herstellung von 2,6-Dichlorbenzoxazol bzw. 2,6-Dichlorbenzthiazol der Formel

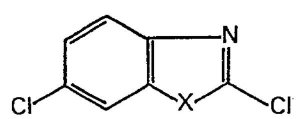

in der X entweder ein Sauerstoff- oder Schwefelatom bedeutet, dadurch gekennzeichnet, dass man das Kalium- oder Natriumsalz des 6-Chlor-2-mercapto-benzoxazols oder -benzthiazols in Anwesenheit eines halogenierten aliphatischen oder aromatischen Kohlenwasserstoffs als Suspendiermittel, gegebenenfalls in Gegenwart eines Katalysators, der Chlorierung unterwirft.

2. Verfahren nach Anspruch 1, dadurch gekennzeichnet, dass als Lösungsmittel Chlorbenzol oder o-Dichlorbenzol verwendet wird.

3. Verfahren nach Ansprüchen 1 und 2, dadurch gekennzeichnet, dass die Chlorierung in Gegenwart von katalytischen Mengen eines N-substituierten Carbonsäureamides durchgeführt wird.

4. Verfahren nach einem der Ansprüche 1, 2

oder 3, dadurch gekennzeichnet, dass im Falle des Thiazols bei erhöhter Temperatur gearbeitet wird.

5. Verfahren nach einem der Ansprüche 1, 2 oder 3, dadurch gekennzeichnet, dass im Falle des Oxazols das Kaliumsalz eingesetzt wird.

**Claims**

1. A process for the manufacture of 2,6-dichlorobenzoxazole or 2,6-dichlorobenzthiazole of the formula

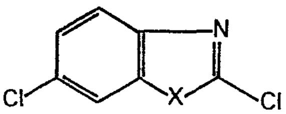

in which X is either oxygen or sulfur, which comprises subjecting the potassium or sodium salt of 6-chloro-2-mercaptobenzoxazole or of 6-chloro-2-mercaptobenzthiazole to chlorination in the presence of a halogenated aliphatic or aromatic hydrocarbon as suspending means and, optionally, a catalyst.

2. A process as claimed in claim 1, wherein the solvent used is chlorobenzene or o-dichlorobenzene.

3. A process as claimed in either of claims 1 and 2, wherein the chlorination is carried out in the presence of catalytic quantities of an N-substituted carboxylic acid amide.

4. A process according to any one of claims 1, 2 or 3, wherein the reaction is carried out at an elevated temperature in the case of the thiazole.

5. A process as claimed in any one of claims 1, 2 or 3, wherein the potassium salt is employed in the case of the oxazole.

**Revendications**

1. Procédé de préparation du dichloro-2,6 benzoxazole ou du dichloro-2,6 benzothiazole répondant à la formule

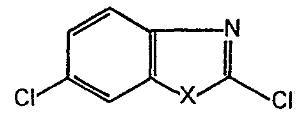

dans laquelle X représente un atome d'oxygène ou de soufre, procédé caractérisé en ce qu'on soumet à la chloration le sel potassique ou sodique du chloro-6 mercapto-2 benzoxazole ou du chloro-6 mercapto-2 benzothiazole, en présence d'un hydrocarbure aliphatique ou aromatique halogéné servant de milieu de suspension et éventuellement en présence d'un catalyseur.

2. Procédé selon la revendication 1, caractérisé en ce qu'on utilise, comme solvant, le chlorobenzène ou l'o-dichloro-benzène.

3. Procédé selon l'une des revendications 1 et 2, caractérisé en ce que la chloration est effectuée en présence de quantités catalytiques d'un carboxamide substitué à l'azote.

4. Procédé selon l'une quelconque des revendications 1, 2 et 3, caractérisé en ce que, dans le cas du thiazole, on opère à une température élevée.

5. Procédé selon l'une quelconque des revendications 1, 2 et 3, caractérisé en ce que, dans le cas de l'oxazole, on utilise le sel de potassium.